# FASCICULE DE BREVET EUROPEEN

(11) **EP 1 174 119 B1**
(45) Date de publication et mention de la délivrance du brevet: **15.01.2003**
(21) Numéro de dépôt: 01121800.5
(22) Date de dépôt: 08.09.2000
(51) Int. Cl.: A61K 7/48

(54) **Composition de nettoyage de la peau**
Hautreinigungsmittel
Skin cleaning composition

(30) Priorité: 18.10.1999 FR 9912971
(43) Date de publication de la demande: 23.01.2002
(62) Demande divisionnaire de: 00402481.6
(73) Titulaire: L'OREAL, 75008 Paris (FR)
(72) Inventeur: Guillou, Véronique, 921600 Antony (FR); Bernard, Pascale, 94370 Sucy en Brie (FR)
(74) Mandataire: Rasson, Catherine

(56) Documents cités:
- EP-A- 0 943 310
- US-A- 5 281 654
- PATENT ABSTRACTS OF JAPAN vol. 018, no. 032 (C-1154), 18 janvier 1994 (1994-01-18) & JP 05 262622 A (NEGAMI KOGYO KK), 12 octobre 1993 (1993-10-12)

## Description

La présente invention se rapporte à l'utilisation d'une composition contenant une dispersion aqueuse de polyuréthanne et une charge pour le nettoyage de la peau, et en particulier comme masque de nettoyage de la peau.

Les produits du type masque de beauté sont bien connus dans le domaine cosmétique. Ils se présentent notamment sous forme de gel, d'émulsion ou de pâte. Il est décrit dans la littérature, par exemple dans "Cosmetic and Toiletry Formulations", Seconde édition, Ernest W. Flick 1992, différentes formules de ce type de masque. Ces masques de beauté peuvent être des masques hydratants sous forme de gel, qui n'ont pas vocation de nettoyage, mais qui procurent à la peau un certain confort.

On connaît également des masques de nettoyage généralement sous forme d'émulsion, contenant en général une charge de type argileux. Toutefois, ces masques ont le désagrément de procurer à la peau une sensation de gras.

Enfin, il existe des masques s'enlevant par pelage, qui sont des compositions filmogènes à base d'alcool polyvinylique. Ces masques de pelage, après application sur le visage, sèchent pour donner un film que l'on retire par arrachage. Pendant le temps de séchage, l'effet occlusif du masque permet à la couche cornée (ou *stratum corneum*) de s'humidifier et de s'assouplir, ce qui peut favoriser la pénétration d'actif, dans le cas où des actifs sont inclus dans la composition. De plus, ces masques, lorsqu'ils sont retirés par arrachage, assurent un "peeling", entraînant notamment les cellules mortes des couches cornées de surface. Ils peuvent également éliminer les comédons et points noirs.

Il est important de pouvoir incorporer des charges telles que le kaolin, dans de telles compositions, afin d'assurer aussi une absorption des composés gras de la surface de l'épiderme et notamment du sébum.

Par ailleurs, l'incorporation de charges permet de renforcer mécaniquement le film et d'opacifier la composition afin de mieux visualiser l'application du film et de faciliter ainsi l'enlèvement dudit film.

Toutefois, il est très difficile d'obtenir une composition stable quand on veut incorporer une quantité suffisante de charges dans une composition de masque pelable à base d'alcool polyvinylique, du fait que ces charges ont tendance à déstabiliser la composition.

Il subsiste donc le besoin d'un masque pelable contenant des charges et ne présentant pas les inconvénients de ceux de l'art antérieur.

La demanderesse a constaté de manière surprenante et inattendue, qu'il était possible d'incorporer des charges, même en quantités importantes, dans des dispersions aqueuses de polyuréthannes (latex), afin d'obtenir des compositions cosmétiques pouvant s'enlever par pelage et pouvant être utilisées comme masques de nettoyage de la peau.

L'invention a donc pour objet l'utilisation cosmétique d'une composition comprenant au moins une dispersion aqueuse de particules de polyuréthanne et au moins une charge choisie parmi la silice, le talc, les argiles, l'oxyde de titane, l'oxyde de zinc, les microsphères, l'amidon, les amidons modifiés et leurs mélanges, pour le nettoyage de la peau du visage.

L'invention a aussi pour objet l'utilisation cosmétique d'une composition comprenant au moins une dispersion aqueuse de particules de polyuréthanne et au moins une charge choisie parmi la silice, le talc, les argiles, l'oxyde de titane, l'oxyde de zinc, les microsphères, l'amidon, les amidons modifiés et leurs mélanges, comme masque de nettoyage de la peau.

La composition utilisée selon l'invention à base d'une dispersion aqueuse de polyuréthanne et d'une charge peut constituer un masque qui a l'avantage de s'étaler facilement sur la peau sans couler, d'être opaque et donc bien visible. En outre, un tel masque permet d'absorber le sébum et autres corps gras de la peau. De plus, cette composition pour masque, après un temps de séchage d'environ 10 à 15 minutes, s'enlève facilement en une seule fois et laisse la peau mate et débarrassée des impuretés.

La viscosité de la composition selon l'invention, mesurée au Rhéomat 180 RM à température ambiante (environ 20-25°C) va de préférence d'environ 5 à 400 Pa.s et de préférence de 10 à 300 Pa.s, car au deçà, la composition est trop fluide et a tendance à couler lors de l'application sur le visage.

La quantité de charges dans la composition de l'invention peut aller par exemple de 1 à 25 % en poids et de préférence de 5 à 20 % en poids par rapport au poids total de la composition.

Comme charges utilisables dans la composition de l'invention, on peut citer la silice ; le talc; les argiles du type montmorillonite, l'hectorite ou la bentonite ; l'oxyde de titane ; l'oxyde de zinc ; les microsphères telles que la poudre de polyméthacrylate de méthyle (MICROPEARL de la Société Matsumoto) et les microsphères creuses expansées (EXPANCEL de la Société Kemanord); l'amidon et les amidons modifiés tels que le produit vendu sous la dénomination DRY FLO par la société National Starch. On peut aussi utiliser un mélange de ces charges.

La dispersion aqueuse de particules de polyuréthanne comprend un ou plusieurs polyuréthannes filmogènes. On peut citer comme polyuréthannes filmogènes, les polyuréthannes anioniques, cationiques, non-ioniques ou amphotères, les polyuréthannes-acryliques, les polyuréthannes-polyvinylpyrrolidones, les polyester-polyuréthannes, les polyéther-polyuréthannes, et leurs mélanges.

Le polyuréthanne peut être, par exemple, un copolymère polyuréthanne, polyurée/uréthanne, ou polyurée, aliphatique, cycloaliphatique ou aromatique, comportant seule ou en mélange :
- au moins une séquence d'origine polyester aliphatique et/ou cycloaliphatique et/ou aromatique, et/ou,
- au moins une séquence siliconée, ramifiée ou non, par exemple polydiméthylsiloxane ou polyméthylphénylsiloxane, et/ou
- au moins une séquence comportant des groupes fluorés.

Le polyuréthanne filmogène peut être choisi notamment parmi les polyester-polyuréthannes et les polyéther-polyuréthannes, et de préférence parmi les polyester-polyuréthannes anioniques, les polyéther-polyuréthannes anioniques et leurs mélanges.

Les polyuréthannes filmogènes utilisables dans la composition de l'invention peuvent être obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.

Avantageusement, on peut utiliser comme dispersion aqueuse de particules de polyuréthanne, une dispersion aqueuse de polyester-polyuréthanne anionique dont la taille des particules de polyuréthanne va de 2 à 100 nm et/ou dont la dureté d'un film obtenu après séchage, durant 24 heures à 30 °C et à 50 % d'humidité relative, d'une couche de 300 µm d'épaisseur (avant séchage) d'une dispersion aqueuse à 28 % de matière sèche desdites particules de polyuréthanne va de 50 à 300 secondes. La dureté du film est mesurée selon la norme ASTM D-43-66, ou la norme NF-T 30-016 (octobre 1981), à l'aide d'un pendule de Persoz.

De telles dispersions aqueuses de polyester-polyuréthanne anionique sont notamment commercialisées sous les dénominations "AVALURE UR 405®, "AVALURE UR 410®", "SANCURE 2060®" par la société Goodrich.

On peut également utiliser comme dispersion aqueuse de particules de polyuréthanne, des dispersions aqueuses de polyéther-polyuréthanne anionique, telles que celles vendues sous les dénominations "SANCURE 878®" par la société Goodrich et "NEOREZ R 970®" par la société ICI.

La dispersion aqueuse de particules de polyuréthanne représente de 5 à 25 % et de préférence de 9 à 20 % en poids de matière sèche par rapport au poids total de la composition.

On peut ajouter à la dispersion aqueuse de polyuréthanne, un ou plusieurs autres polymères filmogènes choisis parmi les polyesters, les polyesters amides, les polyesters à chaîne grasse, les polyamides, les résines époxyesters, les polymères filmogènes radicalaires, les polymères d'origine naturelle, et leurs mélanges.

Les polyesters peuvent être obtenus, de façon connue, par polycondensation de diacides aliphatiques ou aromatiques avec des diols aliphatiques ou aromatiques ou des polyols. Comme diacides aliphatiques, on peut utiliser l'acide succinique, l'acide glutarique, l'acide adipique, l'acide pimélique, l'acide subérique ou l'acide sébacique. Comme diacides aromatiques, on peut utiliser l'acide téréphtalique ou l'acide isophtalique, ou bien encore un dérivé tel que l'anhydride phtalique. Comme diols aliphatiques, on peut utiliser l'éthylène glycol, le propylène glycol, le diéthylène glycol, le néopentyl glycol, le cyclohexane diméthanol, le 4,4'-(1-méthylpropylidène)bisphénol. Comme polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamines, on peut utiliser l'éthylènediamine, l'hexaméthylène-diamine, la méta- ou para-phénylènediamine. Comme aminoalcools, on peut utiliser la monoéthanolamine. Comme monomères porteurs de groupement anionique, pouvant être utilisés lors de la polycondensation, on peut citer par exemple l'acide diméthylol propionique, l'acide trimellitique ou un dérivé tel que l'anhydride trimellitique, le sel de sodium de l'acide sulfo-3 pentanediol, le sel de sodium de l'acide 5-sulfo 1,3-benzène dicarboxylique.

Les polyesters à chaîne grasse peuvent être obtenus par l'utilisation de diols à chaîne grasse lors de la polycondensation.

Les résines époxyesters peuvent être obtenues par polycondensation d'acides gras avec un condensat aux extrémités α, ω - diépoxy.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes radicalaires peuvent être notamment des polymères ou des copolymères, acryliques et/ou vinyliques. On utilise de préférence des polymères radicalaires anioniques. Comme monomère porteur de groupement anionique pouvant être utilisé lors de la polymérisation radicalaire, on peut citer l'acide acrylique, l'acide méthacrylique, l'acide crotonique, l'anhydride maléique, l'acide acrylamido-2 méthyl-2 propane sulfonique.

Les polymères acryliques peuvent résulter de la copolymérisation de monomères choisis parmi les esters et/ou les amides de l'acide acrylique ou de l'acide méthacrylique. Comme exemple de monomères de type ester, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle. Comme exemple de monomères de type amide, on peut citer le N-t-butyl acrylamide et le N-t-octyl acrylamide.

On utilise de préférence des polymères acryliques obtenus par copolymérisation de monomères à insaturation éthylénique contenant des groupements hydrophiles, de préférence de nature non ionique, tels que l'acrylate d'hydroxyéthyle, l'acrylate de 2-hydroxypropyle, le méthacrylate d'hydroxyéthyle, le méthacrylate de 2-hydroxypropyle.

Comme polymère acrylique filmogène utilisable dans la composition de l'invention, on peut citer ceux vendus sous les dénominations NEOCRYL XK-90®, NEOCRYL A-1070®, NEOCRYL A-1090®, NEOCRYL BT-62®, NEOCRYL A-1079®, NEOCRYL A-523® par la société Zeneca, DOW LATEX 432® par la société Dow Chemical, JONCRYL SCX8211 par la société Johnson Polymer.

Les polymères vinyliques peuvent résulter de l'homopolymérisation ou de la copolymérisation de monomères choisis parmi les esters vinyliques, le styrène ou le butadiène. Comme exemple d'esters vinyliques, on peut citer l'acétate de vinyle, le néodécanoate de vinyle, le pivalate de vinyle, le benzoate de vinyle et le t-butyl benzoate de vinyle.

On peut également utiliser des copolymères acryliques/silicones, ou encore des copolymères nitrocellulose/acryliques.

Les polymères d'origine naturelle, éventuellement modifiés, peuvent être choisis parmi la résine shellac, la gomme de sandaraque, les dammars, les élémis, les copals, les dérivés cellulosiques, et leurs mélanges.

La quantité de polymère(s) filmogène(s) autre(s) que le polyuréthanne, peut aller par exemple de 0 à 50 % en poids de matière sèche par rapport au poids de matière sèche de polyuréthanne, et de préférence de 10 à 40 % en poids de matière sèche par rapport au poids de matière sèche de polyuréthanne. Ainsi, le rapport en poids polyuréthanne / autre polymère filmogène peut aller de 100/0 à 50/50 et de préférence de 90/10 à 60/40.

La composition de l'invention utilisée selon l'invention et destinée à une application topique contient un milieu physiologiquement acceptable, c'est-à-dire compatible avec la peau y compris le cuir chevelu, les muqueuses et/ou les yeux.

La composition de l'invention peut contenir également des additifs habituels dans le domaine cosmétique, tels que les actifs, les conservateurs, les gélifiants, les plastifiants, les antioxydants, les solvants, les tensioactifs, les parfums, les absorbeurs d'odeur, les agents antimousse et les matières colorantes, dans la mesure où l'additif ne déstabilise pas la composition ni l'actif si un actif est présent dans la composition. Les quantités de ces différents additifs sont celles classiquement utilisées dans les domaines considérés, et par exemple de 0,01 à 30 % du poids total de la composition.

Comme gélifiants, on peut citer en particulier les gélifiants hydrophiles tels que :
- les dérivés cellulosiques (carboxyméthylcellulose, hydroxyéthylcellulose, hydroxypropylméthylcellulose) ;
- les gommes naturelles telles que les gommes de xanthane, de guar, de caroube, les carraghénanes ;
- les polymères polycarboxyvinyliques du type Carbomer, tels que ceux vendus par la société Goodrich sous les dénominations Carbopol 940, 951, 980, ou par la société 3V-Sigma sous la dénomination Synthalen K ou Synthalen L ;
- les copolymères acryliques tels que les copolymères d'acrylates/alkylacrylates vendus sous les dénominations Pemulen par la société Goodrich ;
- les polyacrylamides et les copolymères d'acrylamide, tels que le produit vendu sous le nom de SEPIGEL 305 par la société SEPPIC (nom CTFA : polyacrylamide/C13-14 isoparaffin/Laureth-7) et le produit vendu sous le nom de HOSTACERIN AMPS par la société HOECHST (nom CTFA : Ammonium polyacryldimethyltauramide) ;
- les polymères associatifs tels que notamment les polyuréthannes associatifs.

Les polyuréthannes associatifs sont des copolymères séquencés non ioniques comportant dans la chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques. En particulier, ces polymères comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter une chaîne hydrocarbonée à un bout ou aux deux bouts d'une séquence hydrophile.

A titre d'exemple, de polymères associatifs utilisables dans l'invention, on peut citer le polymère C₁₆-OE₁₂₀-C₁₆ vendu par la société Hüls sous le nom Sérad FX1100 (molécule à fonction uréthanne et poids moléculaire moyen en poids de 1300, OE étant un motif oxyéthyléné). Comme polymère associatif, on peut aussi utiliser aussi le Rhéolate 205 à fonction urée vendu par la société Rheox ou encore le Rhéolate 208 ou 204. Ces polyuréthannes associatifs sont vendus sous forme pure.

Le produit DW 1206B de chez Rôhm & Haas à chaîne alkyle en C₂₀ et à liaison uréthane, vendu à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Sérad FX1010, le Sérad FX1035 et le Serad 1070 vendus par la société Hüls, le Rhéolate 255, le Rhéolate 278 et le Rhéolate 244 vendus par la société Rheox, les produits DW 1206F et le DW 1206J, ainsi que l'Acrysol RM 184 ou l'Acrysol 44 de la société Röhm & Haas, ou bien encore le Borchigel LW 44 de la société Borchers.

Comme plastifiants, on peut citer par exemple l'adipate de diisobutyle, l'ester de l'acide tertio-butylique et du triméthyl-2,2,4 pentane-diol-1,3, l'adipate de diéthyle, le phtalate de diéthyle, le phtalate de dibutyle, le phtalate de diocyle, le phtalate de butyle et de 2-éthyl hexyle, le sébacate de diméthyle, le sébacate de dibutyle, le stéarate d'éthyle, le palmitate de 2-éthyl hexyle, le dipropylène glycol de diméthyl éther, et leurs mélanges.

Le plastifiant peut être présent dans la composition selon l'invention en une teneur allant par exemple de 0,05 à 2 %, et mieux de 0,1 % à 1,5 % en poids par rapport au poids total de la composition.

Comme solvants ou agents de coalescence, on peut citer par exemple le n-butyl éther de dipropylèneglycol, le n-butyl éther de propylène glycol (PPG-2 butyl ether), les alcools inférieurs comportant de 1 à 4 atomes de carbone tels que l'éthanol, et leurs mélanges. La quantité de solvant dans la composition selon l'invention peut aller par exemple de 1 à 25 % et mieux de 5 à 20 % en poids par rapport au poids total de la composition.

Comme tensioactifs, on peut citer par exemple les diméthicone copolyols tels que le produit commercialisé sous la dénomination Tegopren 5878 par la société Goldschmidt. La quantité de tensioactif dans la composition selon l'invention peut aller par exemple de 0,05 à 2 % et mieux de 0,2 à 1 % en poids par rapport au poids total de la composition.

Comme actifs utilisables dans la composition de l'invention, on peut citer les polyols tels que le propylèneglycol, le dipropylèneglycol, le butylène-1,3 glycol, la glycérine, la polyglycérine et le sorbitol ; les sucres tels que le glucose et le saccharose ; les gluconates tels que le gluconate de magnésium ou de zinc ; les oligo-éléments.

Ainsi qu'indiqué ci-dessus, la composition cosmétique de l'invention, comprenant au moins une dispersion aqueuse de particules de polyuréthanne et au moins une charge, peut constituer notamment un produit de nettoyage de la peau sous forme de masque, destiné aux soins du visage, d'une partie du visage ou du cou, afin notamment de nettoyer la peau en profondeur, par exemple par élimination des cellules mortes de la couche cornée de surface ou par élimination des corps gras présents en excès à la surface de la peau (le sébum par exemple), de raffermir la peau, de l'adoucir et/ou de lui faire subir un traitement particulier.

L'invention a également pour objet un procédé cosmétique de nettoyage de la peau, caractérisé par le fait que l'on applique sur la peau une composition comprenant au moins une dispersion aqueuse de particules de polyuréthanne et au moins une charge choisie parmi la silice, le talc, les argiles, l'oxyde de titane, l'oxyde de zinc, les microsphères, l'amidon, les amidons modifiés et leurs mélanges.

L'exemple ci-après de compositions selon l'invention est donné à titre d'illustration et sans caractère limitatif. Les quantités y sont données en % en poids, sauf mention contraire.

### Exemple

- Dioxyde de titane 1,2 %
- Kaolin 8 %
- Dispersion aqueuse de polyester-polyuréthane anionique
   à 28 % de matière sèche (Avalure UR 405 de Goodrich)
   44,4 % (soit 12,43 % de matière sèche)
- Ammonium styrene/acrylates copolymer
   (Joncryl SCX8211 à 44% de M.A. de Johnson Polymer) 14,2 %
- Sodium maleic anhydride/di-isobutylene copolymer
   (dispersant) (Orotan 731 DP de Rohm & Haas) 0,03 %
- Polyuréthanne (Borchigel LW44 de Borchers) 1,4 %
- PPG-9 Dimethicone Acrylates copolymer (antimousse) 0,18 %
- Gomme de xanthane 2 %
- Conservateur 0,3 %
- Dimethicone copolyol (Tegopren 5878 de Goldschmidt) 0,3 %
- Plastifiant 2 %
- PPG-2 butyl ether 1,3 %
- Ethanol 12,95 %
- Glycérine 5,3 %
- Eau qsp 100 %

On obtient une composition ayant une viscosité au temps zéro (T0) de 19 Pa.s (190 poises) mesurée avec un RHEOMAT 180 RM, mobile 4, à 20°C. Après 10 minutes, la viscosité s'est abaissée à 4,8 Pa.s (48 poises).

Cette composition s'applique comme un masque et s'élimine par arrachage après un certain temps de pause (environ 10 minutes) en laissant une peau douce et nettoyée.

## Revendications

1. Utilisation cosmétique d'une composition comprenant au moins une dispersion aqueuse de particules de polyuréthanne et au moins une charge choisie parmi la silice, le talc, les argiles, l'oxyde de titane, l'oxyde de zinc, les microsphères, l'amidon, les amidons modifiés et leurs mélanges, pour le nettoyage de la peau du visage.

2. Utilisation cosmétique d'une composition comprenant au moins une dispersion aqueuse de particules de polyuréthanne et au moins une charge choisie parmi la silice, le talc, les argiles, l'oxyde de titane, l'oxyde de zinc, les microsphères, l'amidon, les amidons modifiés et leurs mélanges, comme masque de nettoyage de la peau.

3. Utilisation selon la revendication 1 ou 2, **caractérisée par le fait que** la charge est présente en une quantité allant de 1 à 25 % en poids par rapport au poids total de la composition.

4. Utilisation selon l'une quelconque des revendications 1 à 3, **caractérisée par le fait que** la dispersion aqueuse de polyuréthanne est choisie parmi les dispersions aqueuses de polyester-polyuréthanne anionique, les dispersions aqueuses de poléther-polyuréthanne anionique et leurs mélanges.

5. Utilisation selon l'une quelconque des revendications 1 à 4, **caractérisée par le fait que** la dispersion aqueuse de particules de polyuréthanne représente de 5 à 25 % en poids de matière sèche par rapport au poids total de la composition.

6. Utilisation selon l'une quelconque des revendications 1 à 5, **caractérisée par le fait que** la composition comprend en outre un polymère filmogène choisi parmi les polyesters, les polyesters amides, les polyesters à chaîne grasse, les polyamides, les résines époxyesters, les polymères filmogènes radicalaires, les polymères d'origine naturelle, et leurs mélanges.

7. Procédé cosmétique de nettoyage de la peau, **caractérisé par le fait que** l'on applique sur la peau, une composition comprenant au moins une dispersion aqueuse de particules de polyuréthanne et au moins une charge choisie parmi la silice, le talc, les argiles, l'oxyde de titane, l'oxyde de zinc, les microsphères, l'amidon, les amidons modifiés et leurs mélanges.

8. Procédé selon la revendication précédente, **caractérisé par le fait que** la charge est présente en une quantité allant de 1 à 25 % en poids par rapport au poids total de la composition.

## Patentansprüche

1. Kosmetische Verwendung einer Zusammensetzung, die mindestens eine wäßrige Dispersion von Polyurethanpartikeln und mindestens einen Füllstoff enthält, der unter Kieselsäure, Talk, Tonen, Titanoxid, Zinkoxid, Mikrokugeln, Starke, modifizierten Stärkeverbindungen und deren Gemischen ausgewählt ist, für die Reinigung der Gesichtshaut.

2. Kosmetische Verwendung einer Zusammensetzung, die mindestens eine wäßrige Dispersion von Polyurethanpartikeln und mindestens einen Füllstoff enthält, der unter Kieselsäure, Talk, Tonen, Titanoxid, Zinkoxid, Mikrokugeln, Stärke, modifizierten Stärkeverbindungen und deren Gemischen ausgewählt ist, als Maske zur Reinigung der Haut.

3. Kosmetische Verwendung nach Anspruch 1 oder 2, **dadurch gekennzeichnet, daß** der Füllstoff in einer Menge von 1 bis 25 Gew.-%, bezogen auf das Gesamtgewicht der Zusammensetzung, vorliegt.

4. Kosmetische Verwendung nach einem der Ansprüche 1 bis 3, **dadurch gekennzeichnet, daß** die wäßrige Polyurethandispersion unter den wäßrigen Dispersionen von anionischem Polyester-Polyurethan, den wäßrigen Dispersionen von anionischem Polyether-Polyurethan und ihren Gemischen ausgewählt ist.

5. Kosmetische Verwendung nach einem der Ansprüche 1 bis 4, **dadurch gekennzeichnet, daß** die wäßrige Dispersion von Polyurethanpartikeln 5 bis 25 Gew.-% Trockensubstanz, bezogen auf das Gesamtgewicht der Zusammensetzung, ausmacht.

6. Kosmetische Verwendung nach einem der Ansprüche 1 bis 5, **dadurch gekennzeichnet, daß** die Zusammensetzung außerdem ein filmbildendes Polymer enthält, das unter den Polyestern, den Polyesteramiden, den Polyestern mit Fettkette, den Polyamiden, den Epoxyesterharzen, den durch radikalische Polymerisation hergestellten filmbildenden Polymeren, den Polymeren natürlicher Herkunft und ihren Gemischen ausgewählt ist.

7. Kosmetisches Verfahren zur Reinigung der Haut, **dadurch gekennzeichnet, daß** auf die Haut eine Zusammensetzung aufgebracht wird, die mindestens eine wäßrige Dispersion von Polyurethanpartikeln und mindestens einen Füllstoff, der unter Kieselsäure, Talk, Tonen, Titanoxid, Zinkoxid, Mikrokugeln, Stärke, modifizierten Stärkeverbindungen und deren Gemischen ausgewählt ist, enthält.

8. Verfahren nach dem vorhergehenden Anspruch, **dadurch gekennzeichnet, daß** der Füllstoff in einer Menge von 1 bis 25 Gew.-%, bezogen auf das Gesamtgewicht der Zusammensetzung, vorliegt.

## Claims

1. Cosmetic use of a composition comprising at least one aqueous dispersion of polyurethane particles and at least one filler chosen from silica, talc, clays, titanium oxide, zinc oxide, microspheres, starch and modified starches, and mixtures thereof, for cleaning facial skin.

2. Cosmetic use of a composition comprising at least one aqueous dispersion of polyurethane particles and at least one filler chosen from silica, talc, clays, titanium oxide, zinc oxide, microspheres, starch and modified starches, and mixtures thereof, as a cleansing mask for the skin.

3. Use according to Claim 1 or 2, **characterized in that** the filler is present in an amount ranging from 1 to 25% by weight relative to the total weight of the composition.

4. Use according to any one of Claims 1 to 3, **characterized in that** the aqueous dispersion of polyurethane is chosen from aqueous dispersions of anionic polyester-polyurethane and aqueous dispersions of anionic polyether-polyurethane, and mixtures thereof.

5. Use according to any one of Claims 1 to 4,
**characterized in that** the aqueous dispersion of polyurethane particles represents from 5 to 25% by weight of solids relative to the total weight of the composition.

6. Use according to any one of Claims 1 to 5, **characterized in that** the composition also comprises a film-forming polymer chosen from polyesters, polyester amides, fatty-chain polyesters, polyamides, epoxy ester resins, radical-mediated film-forming polymers and polymers of natural origin, and mixtures thereof.

7. Cosmetic process for cleansing the skin, **characterized in that** a composition comprising at least one aqueous dispersion of polyurethane particles and at least one filler chosen from silica, talc, clays, titanium oxide, zinc oxide, microspheres, starch and modified starches, and mixtures thereof, is applied to the skin.

8. Process according to the preceding claim, **characterized in that** the filler is present in an amount ranging from 1 to 25% by weight relative to the total weight of the composition.
